# EUROPEAN PATENT APPLICATION

(11) **EP 4 769 424 A1**
(43) Date of publication of application: **01.07.2026**
(21) Application number: 25227899.9
(22) Date of filing: 31.12.2025
(51) Int. Cl.: G16H 20/40, G16H 30/40, G16H 40/60, G16H 40/67

(54) **SYSTEMS AND METHODS FOR GENERATING MEDICAL AGENTS, AND MEDICAL SYSTEMS**

(30) Priority: 31.12.2024 CN 202412000046
(71) Applicant: Shanghai United Imaging Healthcare Co., Ltd., Shanghai 201807 (CN)
(72) Inventor: RONG, Chengcheng, Shanghai, 201807 (CN)
(74) Representative: Wang, Bo

(57) **Abstract**

Embodiments of the present disclosure provide a medical agent generation system, a medical system, and a medical agent generation method. The system includes an agent platform. The agent platform is deployed on a cloud platform. The agent platform is configured to generate and/or edit a medical agent based on input information of a user. The medical agent is exported to a medical system for execution, and/or the medical agent is imported from the medical system for editing.

## Description

### CROSS-REFERENCE TO RELATED APPLICATIONS

This application claims priority to the Chinese Patent Application No. 202412000046.X, filed on December 31, 2024, the contents of which are hereby incorporated by reference.

### TECHNICAL FIELD

The present disclosure relates to a medical field, and in particular to a medical agent generation system, a medical system, and a medical agent generation method.

### BACKGROUND

The rapid advancement of medical artificial intelligence and large language model technologies has led to a growing number of medical systems adopting intelligent algorithms to improve the efficiency and automation of clinical tasks such as medical.

However, due to the high sensitivity of medical data, healthcare institutions generally adhere to the principle of "keeping data within the hospital," deploying software systems only on internal networks. Such on-premises deployment prevents the direct use of agile development tools based on public cloud platforms, which conflicts with the cloud software service model that emphasizes rapid iteration and agile updates. As a result, hospitals are unable to fully benefit from cloud services. Moreover, developing software functionalities requires professional programming skills. Although large-scale model-based programming assistants have begun to lower the technical barriers, clinical users-such as physicians and medical technicians-still find it difficult to directly create functional modules that meet their needs. These coding assistants typically generate low-level program code, which is not user-friendly. Therefore, low-code technology is needed to provide coarse-grained functional modules that can be automatically assembled through prompts to accomplish specific data processing tasks.

Therefore, it is desired to provide a system and a method for generating a medical agent, and a medical system, which is enable intelligent healthcare workflows while ensuring the security of medical data.

### SUMMARY

One or more embodiments of the present disclosure provide a medical agent generation system. The medical agent generation system includes an agent platform deployed on a cloud platform. The agent platform is configured to generate and/or edit a medical agent based on input information of a user. The medical agent is exported to a medical system for execution, and/or the medical agent is imported from the medical system for editing.

One or more embodiments of the present disclosure provide a medical system. The medical system deployed at a medical institution. The medical system is configured to import an medical agent. The medical system is configured to execute the medical agent based on local clinical data of the medical institution.

One or more embodiments of the present disclosure provide a medical agent generation method. The method is implemented on a medical agent generation system. The method includes generating and/or editing a medical agent based on input information of a user. The method further includes exporting the medical agent to a medical system for execution, and/or importing the medical agent from the medical system for editing.

Additional features will be partly explained in the description below, and will become apparent to those skilled in the art by reviewing the following content and drawings, or through the understanding of production or operation examples. The features of the embodiments described in this specification can be achieved and obtained by practicing or using various aspects of the methods, tools, and combinations detailed in the examples below.

### BRIEF DESCRIPTION OF THE DRAWINGS

The present disclosure will be further described by way of exemplary embodiments. These exemplary embodiments will be described in detail with reference to the accompanying drawings. These embodiments are not limiting. In these embodiments, the same reference numerals refer to the same structures, where:
FIG. 1 is a schematic diagram illustrating a medical agent generation system according to some embodiments of the present disclosure.
FIG. 2 is a schematic diagram illustrating an exemplary structure of the agent platform according to some embodiments of the present disclosure.
FIG. 3 is a schematic diagram illustrating executing an medical agent according to some embodiments of the present disclosure.
FIG. 4 is a schematic diagram illustrating an exemplary process for generating an medical agent according to some embodiments of the present disclosure.
FIG. 5 is a schematic diagram illustrating an exemplary process for generating an medical agent according to other embodiments of the present disclosure.
FIG. 6 is a schematic diagram illustrating an exemplary process for executing an medical agent according to some embodiments of the present disclosure.
FIG. 7 is a schematic diagram illustrating an exemplary process for controlling a treatment couch according to some embodiments of the present disclosure.
FIG. 8 is a schematic diagram illustrating an exemplary process for controlling a multi-leaf collimator to adjust a radiation field shape according to some embodiments of the present disclosure.
FIG. 9 is a schematic diagram illustrating an exemplary process for generating an adaptation prompt of an medical agent according to some embodiments of the present disclosure.
FIG. 10 is a schematic diagram illustrating an exemplary process for determining an execution strategy of an medical agent according to some embodiments of the present disclosure.
FIG. 11 is a schematic diagram illustrating an exemplary process for applying a medical agent according to other embodiments of the present disclosure.
FIG. 12 is a schematic diagram of a medical system architecture according to some embodiments of the present disclosure.

### DETAILED DESCRIPTION

To more clearly illustrate the technical solutions in the embodiments of the present disclosure, the accompanying drawings required for describing the embodiments are briefly described below. Obviously, the accompanying drawings in the following description are merely some examples or embodiments of the present disclosure. For a person of ordinary skill in the art, the present disclosure may be applied to other similar scenarios based on these accompanying drawings without making creative efforts. Unless obvious from the context or otherwise indicated by the context, the same reference numerals in the drawings refer to the same structures or operations.

It should be understood that the terms "system", "device", "unit", and/or "module" used herein are a method for distinguishing components, elements, parts, portions, or assemblies of different levels. However, if other terms can achieve the same purpose, the aforementioned terms may be replaced by other expressions.

As used in the present disclosure and the claims, unless the context clearly indicates an exception, the terms "a", "an", "one", and/or "the" are not limited to the singular form and may include the plural form. Generally, the terms "include" and "comprise" merely indicate that the identified steps and elements are included. These steps and elements do not constitute an exclusive list, and a method or device may also include other steps or elements.

Flowcharts are used in the present disclosure to illustrate operations performed by systems according to the embodiments of the present disclosure. It should be understood that the preceding or following operations are not necessarily performed in exact sequence. On the contrary, the steps may be processed in reverse order or simultaneously. Meanwhile, other operations may be added to these processes, or one or more steps may be removed from these processes.

In the medical field, due to the security and privacy of medical data, medical systems used by medical institutions typically employ traditional upgrade or installation methods. Specifically, during upgrades or installations, users provide vendors with functional requirements or specifications, and software vendors update or develop medical software based on these functional requirements. The updated or developed software is then stored in forms such as a USB drive or a CD and delivered to users for installation on a workstation or medical equipment within the medical system.

However, when software vendors perform upgrades or development based on users' described functional requirements, there may be gaps in understanding or constraints related to product definition strategies, which could result in failed software upgrades or development. Additionally, in the medical field, functional requirements are often associated with the use of clinical data. Medical institutions may also need to provide the clinical data to software vendors so that the software vendors may test the upgraded or developed software system based on the clinical data. However, the clinical data is considered sensitive and private, and transferring the clinical data to software vendors poses risks of data leakage.

To provide convenience for updating medical systems while ensuring the privacy of clinical data, embodiments of the present disclosure provide a medical agent generation system, a medical system, and a medical agent generation method.

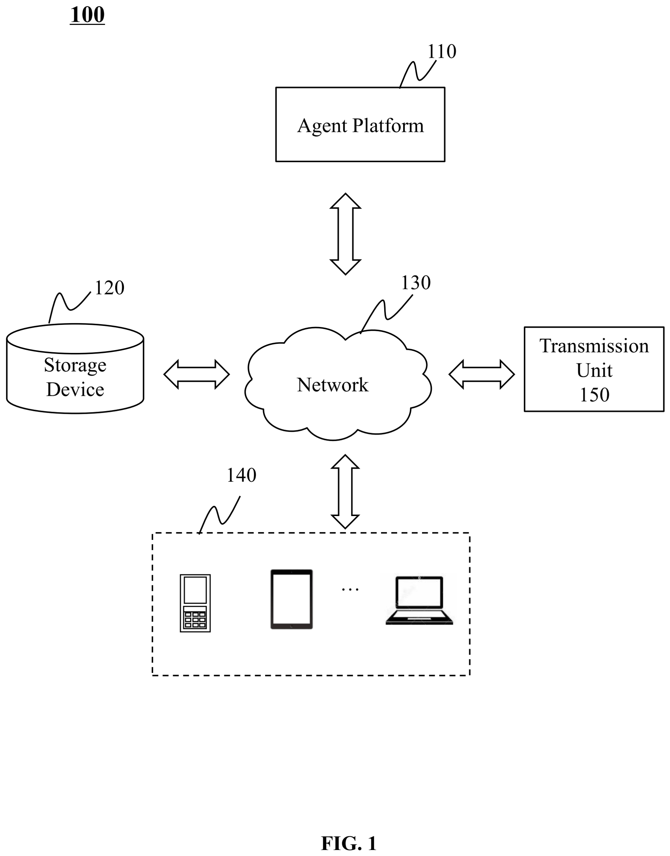

FIG. 1 is a schematic diagram illustrating a medical agent generation system according to some embodiments of the present disclosure. As shown in FIG. 1, the medical agent generation system 100 includes an agent platform 110 deployed on a public cloud, a storage device 120, a network 130, and a terminal 140.

The agent platform 110 refers to a development environment for generating an agent. In some embodiments, the agent platform 110 is deployed on a cloud platform. For example, the agent platform 110 may be deployed on a public cloud, a private cloud, a hybrid cloud, or other environments.

In some embodiments, the agent platform 110 may be a public or private cloud platform. The agent platform 110 includes a platform configured to help enterprises and individuals quickly develop and optimize the agent. The agent platform 110 may provide an open Application Programming Interface (API) and tools to assist users in developing corresponding function modules or applications to build the agent. The agent platform 110 may include a storage-type cloud platform focusing on data storage, a computing-type cloud platform focusing on data processing, a comprehensive cloud computing platform balancing computing and data storage and processing, or the like.

In some embodiments, the agent platform 110 includes a medical functional knowledge base and a preset component library.

The preset component library refers to a collection of predefined functional components. The predefined functional components refer to pre-packaged, and reusable standardized modules. Each predefined functional component in the preset component library encapsulates one or more functions or operational processes related to medical processes. For example, the medical processes include radiotherapy processes. Functions or operational processes related to the radiotherapy processes include an image preprocessing, a structural contour recognition, a dose calculation, a parameter optimization, a result visualization, etc. Each predefined functional component in the preset component library has input/output formats and interface definitions.

In some embodiments, the agent platform 110 may be pre-configured with a component information directory. The component information directory records corresponding functions and storage locations of each component to facilitate querying corresponding preset components. The preset component library includes an image diagnosis function knowledge base, a medical report generation component library, a diagnostic assistance knowledge base, or the like.

The medical functional knowledge base refers to a database that stores professional knowledge in a medical field. For example, the medical functional knowledge base includes contents such as standard processes of medical tasks, operating rules, terminology definitions, equipment capability mapping, interface specifications, etc.

In some embodiments, the agent platform 110 is configured to generate an agent based on input information of a user, the medical functional knowledge base, and the preset component library.

The input information refers to data input by a user to the agent platform 110. For example, the input information includes medical task descriptions in the form of natural language text. In some embodiments, the input information may be input by the user through a visual interface of a terminal 140. In some embodiments, the input information may also be information automatically generated by a computer, or information stored in other devices or specified storage locations (e.g., the storage device 120), and no limitation is imposed thereon. Merely by way of example, the user may input the information based on requirements, or write the requirements into preset documents or tables and store the documents or the tables in a specified storage location. Then, the information is read by the agent platform 110. The requirements refer to requirement information or operation information of the user for the medical agent. For example, the requirements include generating a medical report, performing segmentation on regions of interest (e.g., tumor target areas, organs of interest) in medical images (e.g., CT images, MRI images, etc.), or the like. The user refers to personnel who generate or use the agent. For example, the user may include doctors, medical technicians, or other medical-related personnel.

The types of input information include, but are not limited to, a text, a voice, or an image, or the like, and no limitation is imposed thereon. For ease of explanation, the embodiments of the present disclosure take the type of input information as text as an example for description.

In some embodiments, the input information includes a time range, a data type, or the like of local clinical data. In some embodiments, the input information further includes a requirement for processing the local clinical data

(e.g., a requirement for comparing a segmentation result and a planning result obtained by processing the local clinical data with a historical segmentation result and a historical planning result of historical medical data respectively), a storage location of the local clinical data, whether comparison objects (e.g., the historical segmentation result and the historical planning result) are historical medical data in a medical database, a display mode of comparison results, an algorithm and an operational process for processing the local clinical data, or other information, and no limitation is imposed thereon. The segmentation result refers to three-dimensional contours or mask data generated after performing segmentation on a region of interest in a medical image. The planning result refers to pre-formulated standard treatment data based on clinical diagnosis, patient conditions, and treatment objectives during a medical planning stage. Taking a radiotherapy planning stage as an example of the medical planning stage, the planning result includes a planned dose distribution, planned beam parameters (e.g., angles, energy, number of sub-fields), a planned DVH curve (Dose-Volume Histogram), or the like. The historical segmentation result refers to an image segmentation result in a historical medical process. The historical planning result refers to standard treatment data in a historical medical process. The segmentation result and the planning result may be determined by the agent. The historical segmentation result and the historical planning result may be pre-stored in a storage device (e.g., the storage device 120). The medical database refers to a database storing a large amount of historical clinical medical data. Data in the medical database may be determined based on historical clinical treatment processes.

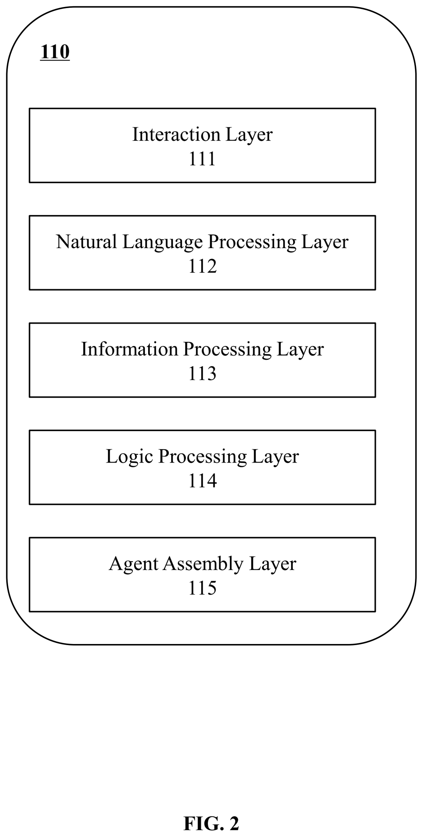

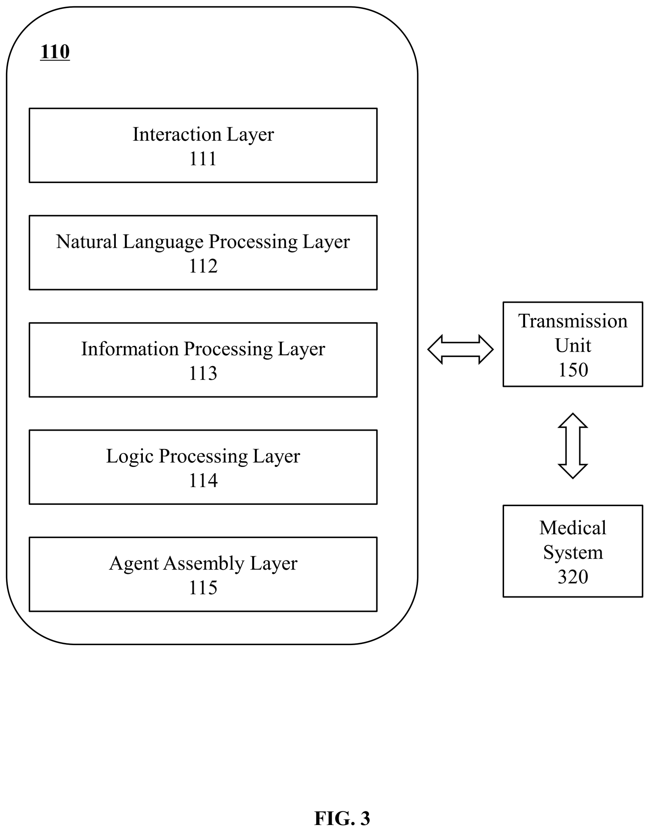

The medical agent refers to a collection of advanced computer programs constructed based on technologies such as natural language processing, machine learning, data analysis, or the like. In some embodiments, the medical agent may also be referred to as an agent, an intelligent agent, or an AI agent. In some embodiments, the medical agent is exported to a medical system for execution, and/or the medical agent is imported from the medical system for editing. More descriptions regarding the execution and editing of the medical agent may be found in FIG. 2, FIG. 3, etc.

The agent may be configured to simulate decision-making processes of humans (e.g., medical experts). The agent is capable of analyzing information, making decisions, and responding by performing actions to achieve specific goals. Merely by way of example, the agent may understand and respond to medical information through technologies such as natural language processing, machine learning, data analysis, or the like, and provide specific services such as diagnostic support, treatment suggestions, and patient care guidance. The agent is capable of continuously learning and adapting to improve accuracy and efficiency in medical decision-making and patient management. The agent may include a combination of one or more software function modules or applications configured to achieve specific goals. The user may input information on the agent platform 110, and the agent platform 110 automatically generates an agent meeting the demands based on the input information. Through the agent platform, the programming technology threshold required for the user to develop the agent may be reduced.

In some embodiments, the agent may be a native agent in the agent platform or a medical system. The agent platform may edit (e.g., add, delete, modify, and/or change part or all of the content of the native agent, or the like) the native agent based on the input information to obtain an agent meeting the demands. Further, there is no need to develop the agent meeting the demands from scratch, and only the native agent needs to be edited, so as to further improve the generation efficiency of the agent. The agent meeting the demands refers to an agent applicable to a specific medical field. For example, the agent meeting the demands may include an agent applied to the radiotherapy field. The native agent refers to an agent pre-deployed (e.g., deployed in the storage device 120) and directly executable.

In some embodiments, the agent platform 110 may select target components from the preset component library based on the input information, to generate and/or edit the agent. Merely by way of example, an application generation network model may be pre-configured in the agent platform 110. The application generation network model may perform feature processing on the input information to identify functional features corresponding to the input information. Then, the agent platform 110 selects the target components from the preset component library based on the functional features to construct and/or edit the target components, so as to obtain the agent.

The application generation network model is a machine learning model, for example, any one or a combination of neural network models, deep neural network models, convolutional neural network models, Transformers, or the like. The feature processing refers to a process of converting original, unstructured input information (e.g., comparing the medical planning result of tumor patient A with historical planning results of medical for other tumor patients in the recent 3 months) into structured data (e.g., keywords, functional tags, or the like) recognizable by the model. The functional features refer to specific functional demands that the agent needs to achieve as described in the input information (which are core outputs after feature processing), for example, "data screening (time range: 3 months, population: tumor patients)", "data comparison (planning results of tumor patient A and historical planning results of medical for other tumor patients)", "result visualization", or the like.

In some embodiments, the agent platform generates the medical agent based on a pipeline layout. The pipeline layout describes an overall process structure and a data flow relationship of the medical agent. In some embodiments, the agent platform 110 is configured to receive the input information. In some embodiments, the agent platform 110 is further configured to determine a data type of local clinical data and a time range of the local clinical data to be extracted from the medical system based on the input information. In some embodiments, the agent platform 110 is further configured to generate function description information for executing the agent. In some embodiments, the agent platform 110 is further configured to generate the pipeline layout based on the data type of the local clinical data, the time range of the local clinical data, the function description information, and a medical functional knowledge base. In some embodiments, the agent platform further includes an interaction layer, a natural language processing layer, an information processing layer, and a logic processing layer. In some embodiments, the agent platform may generate the pipeline layout through the interaction layer, the natural language processing layer, the information processing layer, and the logic processing layer, and further generate the medical agent. More descriptions regarding related content may be found in FIG. 2.

The storage device 120 is capable of storing data, instructions, and/or any other information. For example, the storage device 120 may store the medical functional knowledge base, the preset component library, or the like. In some embodiments, the storage device 120 may include mass storage devices, removable storage devices, volatile read-write memory, Read-Only Memory (ROM), or the like, or any combination thereof. In some embodiments, the storage device 120 may be deployed on a cloud platform.

The network 130 may connect various components in the system 100 for generating the medical agent and/or connect other components outside the system 100 for generating the medical agent. In some embodiments, one or more components (e.g., the agent platform 110, the storage device 120, the terminal 140, or the like) of the system 100 for generating the medical agent may achieve connection and/or communication with each other through the network 130.

The terminal 140 is configured to provide functional components related to user interaction and implement user interaction functions (e.g., provide or display information and data for the user). Merely by way of example, the terminal 140 may be one or any combination of a mobile device, a tablet computer, a laptop computer, a desktop computer, or other devices with input and/or output functions. Merely by way of example, the output functions include, but are not limited to, one or a combination of audio output such as voice, display screen, haptic transmission such as vibration, electromagnetic wave signals such as light, or the like. Merely by way of example, the input functions may include, but are not limited to, one or a combination of keyboard input, touch screen input, voice input, motion event input such as device tilting/shaking/rotating/swinging, electromagnetic wave signal input such as light, or the like.

In some embodiments, the terminal 140 includes a visual interface, and the visual interface may provide display and input functions for the user. For example, the visual interface may display the agent. As another example, the user may input information through the visual interface.

In some embodiments, the medical agent generation system 100 may further includes a transmission unit 150. The transmission unit 150 refers to a functional module for implementing bidirectional data transmission between the agent platform 110 and a medical system. In some embodiments, the transmission unit 150 may be a portion of the network 130.

In some embodiments, the transmission unit 150 is configured to export the medical agent to the medical system for execution, and/or import the medical agent from the medical system for editing. More descriptions regarding the medical system importing the medical agent into the agent platform 110 through the transmission unit 150 for editing may be found in FIG. 2. More descriptions regarding exporting the medical agent to the medical system for executing the medical agent may be found in FIG. 3.

It should be noted that the system 100 for generating the medical agent is merely provided for illustrative purposes and not intended to limit the scope of the present disclosure. For a person of ordinary skill in the art, various modifications or changes may be made according to the description of the present disclosure. For example, the system 100 for generating the medical agent may implement similar or different functions on other devices. However, these modifications and changes will not depart from the scope of the present disclosure.

FIG. 2 is a schematic diagram illustrating an exemplary structure of the agent platform according to some embodiments of the present disclosure. As shown in FIG. 2, the agent platform 110 includes an interaction layer 111, a natural language processing layer 112, an information processing layer 113, and a logic processing layer 114.

The interaction layer 111 refers to a medium for providing interaction between a user and the agent platform 110. In some embodiments, the interaction layer 111 is configured to receive the input information. For example, the interaction layer 111 is configured to receive input information from the user.

Merely by way of example, the interaction layer 111 may be a visual interface or a software program. The interaction layer 111 may receive information input by the user or respond to operations of the user on hardware or software, and provide responses based on the input information and operations to achieve dialogue or interaction with the user.

It should be noted that the interaction layer 111 is a medium for the user to provide input information to the agent platform 110. In practical scenarios, the agent platform 110 usually needs to understand the input information input by the user, then feed back reply information based on the understood input information, and display the reply information in the interaction layer 111. Further, interaction with the user is achieved. Alternatively, generation and/or editing of the agent is performed based on the understood input information. For example, as a non-limiting example of the user providing input information to the agent platform 110, the user may input descriptions (e.g., text descriptions, voice descriptions, or the like) of functional demands for the agent, or input operations (e.g., selecting entries, dragging icons, or the like) on patterns or entries representing respective functions supported by the agent, so that the user can select desired functions of the agent and thus customize and design the desired agent.

The natural language processing layer 112 refers to an architecture for parsing and processing the input information of the user. In some embodiments, the natural language processing layer 112 may process medical terms in the input information. For example, the natural language processing layer 112 may identify professional medical terms (e.g., lung cancer, chemotherapy, DICOM images, blood glucose control effects, or the like) in the input information, and unify synonymous terms (e.g., radiotherapy, radiation therapy, RT, or the like) into standard tags, or the like. For clarity of description, some embodiments of the present disclosure are described with reference to radiotherapy for a patient.

In some embodiments, the natural language processing layer 112 is configured to determine a data type of local clinical data and a time range of the local clinical data to be extracted from the medical system based on the input information. For example, the natural language processing layer 112 may perform semantic parsing on the input information, and determine the data type and the time range of the local clinical data to be extracted from the medical system based on results of the semantic parsing.

The data type refers to a specific data category of the local clinical data involved when executing the agent. For example, the data type includes medical record data of patients, radiotherapy segmentation results, dose distribution data, or the like.

The time range refers to a time interval corresponding to the local clinical data that the user expects the agent to analyze. For example, the time range includes treatment records within the time interval (e.g., recent three months, or the like). More descriptions regarding the medical system and the local clinical data may be found in FIG. 3.

In some embodiments, the natural language processing layer 112 may parse the input information of the user through a preset semantic recognition rule, automatically extract the data type (e.g., medical records, images, structural contours, dose plans, or the like) and time range (e.g., "in the recent six months", "data in 2022", or the like), and convert the data type and the time range into structured fields to facilitate subsequent processing.

The information processing layer 113 refers to an architecture configured to determine function description information associated with execution of the agent based on the input information. In some embodiments, the information processing layer 113 is configured to generate the function description information for executing the agent. The function description information refers to information capable of being understood by the medical agent after the information processing layer 113 processes and converts the input information. The agent may interact with the user based on the understood function description information. For example, the function description information includes data sources (e.g., Picture Archiving and Communication Systems (PACS), Treatment Planning Systems (TPS), Electronic Medical Record (EMR) systems, or the like), invoked algorithms (e.g., tumor segmentation algorithms, dose prediction models, or the like), processing logics (e.g., batch processing/screening/result output formats), or the like. The function description information is configured to support process design and component scheduling of the agent.

A language dialogue model trained based on dialogue texts may be pre-configured in the information processing layer 113. The language dialogue model is configured to process the input information input by the user to achieve interaction with the user based on the understood input information.

As an example, an execution scenario of the interaction layer 111, the natural language processing layer 112, and the information processing layer 113 may be as follows: the interaction layer 111 receives input information input by the user and sends the input information to the natural language processing layer 112 and the information processing layer 113. The natural language processing layer 112 determines a data type and a time range of local clinical data to be extracted from the medical system based on the input information. The information processing layer 113 processes and converts the input information to obtain converted information, and outputs corresponding reply information to the interaction layer 111 for display based on the converted information. Then, the interaction layer 111 receives input information input by the user again and sends the input information to the information processing layer 113. After the interaction as described above, the information processing layer 113 may extract information from the input information from multiple inputs from the user to obtain function description information.

Merely by way of example, take a case where the input information includes a type of clinical function to be used. When the interaction layer 111 receives the input information of "having a function of storing clinical data", the interaction layer 111 sends the input information to the natural language processing layer 112 and the information processing layer 113. After processing the input information, the information processing layer 113 may output reply information of "what are the types of clinical data to be stored" to the interaction layer 111, or display input boxes or selection lists corresponding to the types of clinical data to be stored in the interaction layer 111. Then, the interaction layer 111 may receive input information input by the user again and send the input information input by the user again to the natural language processing layer 112 and the information processing layer 113. The natural language processing layer 112 determines the data type of the local clinical data to be extracted from the medical system based on the input information input by the user again. The information processing layer 113 may determine the function description information as "storing clinical data" based on the input information input by the user again. The reply information output by the information processing layer 113 may further include "what is the time range of the clinical data to be stored". When the user inputs the time range, the natural language processing layer 112 determines the time range of the local clinical data to be extracted from the medical system based on the time range input by the user.

The function description information may be configured to describe one or more functions to be implemented by the finally constructed agent. It can be understood that the preset component library includes a plurality of components as described above, and each component is configured to identify one function or one operational process. Based on this, a plurality of target components required for constructing the agent may be determined based on the function description information.

In some embodiments, the function description information may be identification information of types such as numbers, letters (configured to identify corresponding functions), or text information, etc.

The logic processing layer 114 refers to an architecture configured to construct a pipeline layout capable of implementing desired functions of the agent based on the function description information.

The pipeline layout may be regarded as a flowchart, a layout, or a model of programming ideas for implementing specific functions, and no limitation is imposed thereon. The pipeline layout may describe an overall process structure and a data flow relationship of the agent.

In some embodiments, the logic processing layer 114 is configured to generate the pipeline layout based on the data type, the time range, the function description information, and the medical functional knowledge base. The data type may be determined based on the natural language processing layer 112. The time range may be determined based on the natural language processing layer 112 or based on user input received by the interaction layer 111. The function description information may be determined based on the information processing layer 113.

The pipeline layout includes a plurality of nodes. Each node in the pipeline layout may correspond to one component, and a connection relationship between each node in the pipeline layout may be regarded as describing a positional relationship between each component and an operational sequence (which may also be regarded as a processing sequence of data during agent operation) during component operation, and no limitation is imposed thereon. A node in the pipeline layout refers to a logical element representing a specific functional unit or an operational step in the pipeline layout.

In some embodiments, the logic processing layer 114 may screen components capable of processing corresponding data formats from the preset component library according to the extracted data type. The logic processing layer 114 may simultaneously set data screening parameters (e.g., start and end times, case scopes, or the like) of the components according to the time range. The logic processing layer 114 may further retrieve functional components matching the function description information from the medical functional knowledge base, and automatically determine an execution sequence and a data transmission path between each component in combination with pre-defined task structures and path rules in the medical functional knowledge base, so as to generate the pipeline layout.

As an example, a trained design generation neural network model may also be pre-configured in the logic processing layer 114. The trained design generation neural network model is configured to automatically generate the pipeline layout based on the function description information.

In some embodiments, the agent platform further includes an agent assembly layer 115.

In some embodiments, the agent assembly layer 115 is configured to generate an agent based on the pipeline layout and the preset component library.

In some embodiments, the agent assembly layer 115 may invoke corresponding functional modules from the preset component library according to task structures and functional nodes defined in the pipeline layout, encapsulate and generate executable scripts, and combine the executable scripts to form the agent.

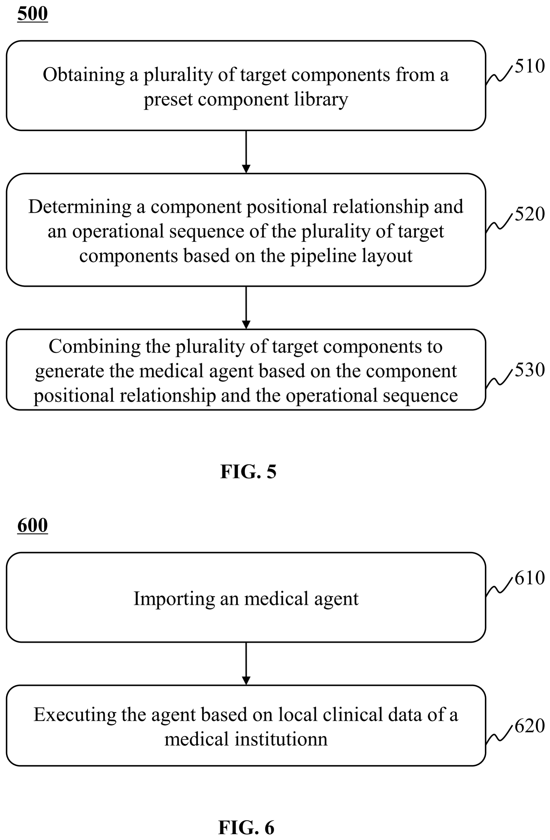

In some embodiments, to generate the agent based on the pipeline layout and the preset component library, the agent assembly layer 115 is further configured to obtain a plurality of target components from the preset component library. Each of the plurality of target components is configured to execute a specific medical subtask. The agent assembly layer 115 is further configured to determine a component positional relationship and an operational sequence of the plurality of target components based on the pipeline layout. The agent assembly layer 115 is further configured to combine the plurality of target components to generate the agent based on the component positional relationship and the operational sequence. More descriptions regarding related content may be found in FIG. 5.

It should be particularly noted that the generated agent needs to be imported into a private medical system for use. Moreover, after being imported, when the agent being operated, the agent usually needs to obtain local clinical data from a medical database in the medical system to implement corresponding functions.

However, due to the privacy of the clinical data, the agent usually cannot directly retrieve the local clinical data from the medical database of the medical system during operation. In some embodiments, the agent assembly layer 115 is further configured to determine a system access interface based on an interface protocol of the medical system, and extract the local clinical data based on the system access interface. More descriptions regarding the medical system and the local clinical data may be found in FIG. 3.

The interface protocol refers to a standardized communication criterion for interaction between the agent and the medical system during execution. The interface protocol includes data access permissions, interaction manners, and the like of the agent in the medical system during operation. The interface protocol may ensure data security in the medical system.

In some embodiments, the interface protocol may be obtained by the user through pre-uploading the interface protocol to the agent platform 110 when constructing the agent on the agent platform 110.

The system access interface refers to a standardized data calling path of the medical system through which the local clinical data is retrieved from the medical system. In some embodiments, the system access interface is generated by the agent assembly layer 115 according to the interface protocol of the medical system. The system access interface may be embedded into the agent in the form of an API call. During execution, the agent may securely retrieve the local clinical data of the medical system through the system access interface. The system access interface may be a pre-configured access interface in the agent platform, or an access interface automatically generated by the agent platform based on the interface protocol, and no limitation is imposed thereon.

Merely by way of example, the agent platform 110 may be configured with access interfaces corresponding to a plurality of types of interface protocols respectively, and then determine the system access interface from the plurality of access interfaces based on the interface protocol of the medical system.

The system access interface allows users, applications (e.g., including the agent or a portion thereof), or other components to interact with the system (e.g., the medical system). The system access interface includes service interfaces that allow the agent to request medical system services (e.g., file operations, process management), database interfaces for accessing databases (e.g., the medical database), network communication interfaces for implementing remote access and inter-service communication, or the like, and no limitation is imposed thereon.

Merely by way of example, when the system access interface is a database interface of the medical database, the agent assembly layer 115 may analyze data of the medical database based on a preset script generation model to generate corresponding SQL query codes and embed the SQL query codes into a script, so as to obtain the aforementioned system access interface.

In some embodiments of the present disclosure, by binding a system access path that complies with the interface protocol during the agent generation phase, automatic insertion of data access logic and secure calling during the execution phase are achieved, which improves portability and controllability of the agent in different hospital deployment environments, and ensures secure isolation and compliant use of clinical data.

In some embodiments, in practical scenarios, due to inaccuracies in the user's description of functions to be implemented by the agent (e.g., the input information cannot accurately express the functions to be implemented), and understanding deviations of the natural language processing layer 112 and/or the information processing layer 113 after processing the input information, the pipeline layout generated by the logic processing layer 114 based on the understood function description information may fail to meet expectations.

In some embodiments, to improve accuracy of the generated pipeline layout, the agent platform 110 is further configured to: display the pipeline layout in a visual interface; edit the pipeline layout based on an editing instruction of the user; perform verification on the agent based on preset test data; and display a verification result in the visual interface.

In some embodiments, the interaction layer 111 of the agent platform 110 may display the pipeline layout in a visual interface (e.g., the visual interface of the terminal 140).

In some embodiments, presenting the pipeline layout to the user through the visual interface facilitates the user to intuitively view execution steps, data flow directions, and logical structures of the agent, thereby facilitating understanding and adjustment.

In some embodiments, the visual interface may provide editing functions for the user to perform editing operations. The editing instruction refers to an instruction for editing and modifying the pipeline layout. The editing instruction includes instructions for adding, deleting, changing, modifying, or the like of components of the pipeline layout. Merely by way of example, the editing instruction may include adding or deleting one or more components, modifying a component relationship between the components, or the like.

In some embodiments of the present disclosure, by displaying the pipeline layout in the visual interface and providing the editing functions, the user is facilitated to make detailed adjustments to the pipeline layout during the process of generating the agent, thereby improving the accuracy of the design of functional components.

In some embodiments, the agent generated by the agent platform 110 may be imported into the medical system for execution, and the agent in the medical system may also be imported into the agent platform 110 for editing. For the native agent imported from the medical system, the agent platform 110 may further display functional components included in the native agent and a component relationship between the functional components in the visual interface of the interaction layer 111. That is, the pipeline layout corresponding to the native agent is displayed. Then, in response to the editing instruction of the user, the agent platform 110 may edit the pipeline layout to obtain an edited pipeline layout, so as to reconstruct the agent.

In some embodiments, after generating the agent, to ensure that the agent can implement corresponding functions, the agent platform 110 may further perform testing on the agent. Specifically, the interaction layer 111 is further configured to perform verification on the agent based on preset test data to obtain a verification result, and display the verification result in the visual interface. The user determines whether to adjust the agent based on the displayed verification result.

The preset test data is standardized case data which does not contain privacy information of a subject and is only used for testing. The subject includes an in-hospital patient, an outpatient, a healthy volunteer, a simulated phantom, a laboratory animal, etc. The term "standardized" refers that a data format, a quality standard, and an annotation standard of the case data are unified. In some embodiments, the preset test data may be stored in a database in the agent platform 110. The database may be divided into a data knowledge base for performing rule verification on the agent and a functional test library for verifying whether the agent completes functions during operation. The data knowledge base may be pre-configured with data for performing rule verification on the agent to verify whether the agent has risks related to data deletion and modification. The functional test library may be configured with data supporting automatic execution of the agent to verify whether the effect of the agent in implementing functions meets expectations.

In some embodiments, the verification result may include a rule verification result from the rule verification and a functional verification result from the functional verification. Both the rule verification result and the functional verification result may be compared with expected verification results respectively to determine whether the construction of the agent meets expectations.

In some embodiments, during the testing process, the agent platform 110 may further display the execution process of the agent on the visual display interface of the interaction layer 111, so as to facilitate the user to intuitively understand the execution process. Further, the user is enabled to further determine whether the construction of the agent meets expectations.

In some embodiments, when data from the data knowledge base and the functional test library is obtained, a query component of a data warehouse may be further configured in the agent platform 110 for data reading. The query component refers to a module for reading, screening, and obtaining data from the data knowledge base or the functional test library (collectively referred to as the data warehouse). The query component may also be configured on the visual display interface of the interaction layer 111 for the user to invoke. Merely by way of example, when the query component is invoked, the visual interface may display a preset query editing area. The agent platform 110 may obtain data from the data warehouse based on query conditions input by the user in the query editing area.

In some embodiments, the interaction layer 111, the natural language processing layer 112, the information processing layer 113, the logic processing layer 113 and the agent assembly layer 115 are modular components of the agent platform 110, i.e., the agent platform 110 is pre-divided into these layers based on the system architecture of the agent platform.

In some embodiments, for any user who constructs an agent on the agent platform 110, the agent platform 110 may further provide functions such as deletion and version update of the agent for the user. A sharing community or an agent store may also be pre-established on the agent platform 110, and each user may selectively put the agent constructed by themselves into the sharing community or the agent store, or allow some users to download and use the agent. In some embodiments of the present disclosure, the agent platform 110 may automatically generate and/or edit the agent based on the input information without requiring the user to understand programming languages or write program codes, thereby improving the generation efficiency of the agent.

FIG. 3 is a schematic diagram illustrating executing a medical agent according to some embodiments of the present disclosure. In some embodiments, as shown in FIG. 3, after generating and/or editing the agent, the agent platform 110 may further export the agent to a medical system 320 through a transmission unit 150 for execution. In some embodiments, the medical system 320 may also import the agent into the agent platform 110 through the transmission unit 150 for editing. More descriptions regarding the medical system 320 importing the medical agent into the agent platform 110 through the transmission unit 150 for editing may be found in FIG. 2.

The medical system 320 refers to a dedicated business system deployed within a medical institution. The medical system 320 has capabilities of importing, verifying, and executing the agent. The medical system 320 may invoke local clinical data to complete medical-related tasks. The medical institution refers to a medical service institution that provides services related to disease diagnosis, treatment and health care. For example, a medical institution includes a hospital, an outpatient department, a clinic, a medical consortium, a regional medical center or other medical service entities.

The local clinical data refers to private medical information stored in a local database of the medical institution. For example, the local clinical data includes patient image data (e.g., CT/CBCT data), treatment records, target area/organ structural contours, radiotherapy dose plans, or the like. The local clinical data only circulates within the medical institution. The local database of the medical institution is also referred to as the medical database.

In some embodiments, the medical system 320 may import and validate the agent. The medical system 320 executes the agent based on the local clinical data of the medical institution.

The medical system 320 may import and/or export the aforementioned agent through a pre-designed plug-in interface to complete the upgrade or update of clinical application functions for local clinical data in the medical system 320.

It should be noted that after importing the agent, to ensure that the agent can operate normally in the medical system 320, the medical system 320 may first perform verification of adaptability, security, and functionality on the agent to determine whether the agent has situations such as viruses, version incompatibility, the medical system 320 lacking controls to support the operation of the agent, or the like during the import process.

In some embodiments, a preset set of vulnerability verification rules may be pre-configured in the medical system 320 to verify whether the agent has viruses. The medical system 320 may determine whether a version of a currently imported agent is a preset latest version based on the vulnerability verification rules. The medical system 320 may determine whether certain functions of the agent may be normally implemented based on the vulnerability verification rules. The medical system 320 may further determine whether the medical system 320 lacks controls for implementing certain functions of the agent based on the vulnerability verification rules.

It may be understood that since the constructed agent performs the aforementioned verification in the medical system 320, none of the test data required for the verification process needs to be sent outside. Further, the risk of data leakage can be avoided.

In some embodiments, after the agent passes the verification, the medical system 320 may execute the agent. In some embodiments, the agent is applied to the medical field. For example, the agent is applied to a radiotherapy field. In some embodiments, the medical system 320 includes a radiotherapy system. The radiation therapy system is a dedicated medical system for performing radiation therapy against malignant tumors. The radiation therapy system may destroy tumor cells and inhibit tumor proliferation by means of ionizing radiation/radioactive rays and the like, while protecting the normal tissues and organs around the lesion to the maximum extent.

In some embodiments, the medical system 320 executes the script and component calling logic included in the agent according to the pipeline layout, loads required patient data from the local clinical database of the medical institution, and completes medical-related tasks including image analysis, determination of parameters (e.g., target area volume, dose distribution, or the like), generation of results (e.g., medical plan evaluation reports, segmentation result comparison charts, etc.), or the like. The component calling logic refers to the component execution sequence and dependency relationships defined according to the nodes in the pipeline layout.

It should be supplemented that since the agent is imported by the user on the public agent platform 110, if the agent is granted permission to access the local database of the medical institution during operation, there may still be a risk that the local clinical data is maliciously read or modified.

In some embodiments, the medical agent is executed on a standalone device or in an isolated environment to further ensure the security of the local clinical data. Data, processes, and the like in the standalone device or the isolated environment are not subject to interference from any other device or environment. The standalone device refers to a terminal with a complete physical hardware architecture and independent operational capability, capable of executing the agent individually. For example, the standalone device may be a dedicated medical terminal, a local server, or a portable medical computing device. The standalone device does not connect to external networks. The standalone device does not rely on external API interfaces, third-party components, or support from distributed architectures. The standalone device depends solely on built-in processors, memory, and storage modules to execute the agent. The local clinical data required during agent execution is obtained through local input (e.g., manual entry by healthcare professionals, direct data acquisition from locally connected medical devices). Data processing and result storage are performed locally without transmitting or synchronizing data externally. In some embodiments, the medical system 320 may execute the agent in isolation during the operation of the agent. That is, the medical system 320 provides the isolated environment for the operation of the agent.

Merely by way of example, to ensure data security, it is crucial to separately isolate the environment in which the agent is executed. The medical system 320 may adopt virtualization technologies, such as creating an independent operating environment through virtual machine technologies (e.g., VMware, VirtualBox, or the like) or container technologies (e.g., Docker, Kubernetes, or the like). Taking container technology as an example, each agent may be deployed in an independent Docker container, and there are strict resource isolation and network isolation mechanisms between containers. That is, data, processes, and the like in the container where one agent is located will not be interfered by agents in other containers or external factors. Therefore, even if a certain agent malfunctions or suffers a security threat (e.g., malicious attacks, or the like), it is difficult to break through the isolation limitations of the container to affect other agents and clinical data in the medical system. Further, the security of clinical data is ensured.

In addition, security protection measures such as Access Control Lists (ACLs) and firewalls are configured at the network level to strictly restrict access to the agent execution environment from external networks. Only authorized terminals (e.g., doctor workstations, computers at nurse stations, or the like) in the medical system 320 are allowed to access according to specified protocols and ports, thereby further enhancing the security of the environment.

In some embodiments, the medical system 320 may obtain target data generated after the agent processes data in a copy database, and display the target data in a visual interface. Finally, in response to a confirmation operation of the user, the medical system 320 stores the target data in the medical database. Data in the copy database is the same as data in the medical database. The copy database refers to a backup of the medical database.

In some embodiments, the medical database may be a database storing data related to the medical field, including, but not limited to, clinical databases for collecting and storing clinical data (e.g., medical record diagnoses, treatment plans, or the like) of patients, genomic databases for storing genomic sequence data and related genetic information, drug databases for storing drug information, and literature databases for storing medical literature, or the like, and no limitation is imposed thereon.

Processing of data in the copy database by the agent includes, but is not limited to, modifying, adding, or deleting data in the copy database, and no limitation is imposed thereon.

It should be noted that a large amount of data usually exists in the medical database. When directly modifying data in the medical database based on the agent, uncontrollable changes may occur to the medical database, increasing the difficulty of maintaining the medical database in the later stage.

Based on this, to protect data in the medical database, when the agent needs to process data in the medical database, the data in the medical database may first be copied to the copy database, so that the data in the copy database is the same as the data in the medical database. Then, the medical system 320 may display the target data generated after the data in the copy database is processed in the visual interface. When the user accepts the modified result, that is, after detecting a confirmation operation of the user, the target data is stored in the medical database. At this time, the original data in the medical database may be deleted or retained, and no limitation is imposed thereon. In addition, when a deletion operation is detected, the medical system 320 does not need to store the target data in the medical database.

In some embodiments, to further ensure the security and privacy of clinical data, when the agent is executed in a separately isolated environment, operations on clinical data may not only meet the aforementioned isolation requirements but also meet requirements of being revocable and auditable.

Merely by way of example, during the execution of the agent, if the user subsequently finds that a previous operation is a misoperation, the agent may respond to the user's operation again to restore the executed clinical data operation, thereby avoiding adverse effects caused by incorrect clinical data operations and ensuring that clinical data is always in an accurate and reliable state.

In some embodiments, when executing the agent, the medical system 320 may send local clinical data to the agent through a system access interface. More descriptions regarding the medical system sending local clinical data to the agent through the system access interface may be found in FIG. 2.

During the execution of the agent, each operation on clinical data may be recorded for subsequent auditing. Further, it is convenient for relevant personnel to verify later, ensuring that the use of clinical data is carried out within a reasonable and legal scope.

Based on the above descriptions, in the present embodiment, to conveniently add new functions to the medical system, a medical agent system is provided. The medical agent system includes an agent platform and a transmission unit. The agent platform may generate and/or edit an agent based on input information. Then, the transmission unit exports the generated or edited agent to the medical system for execution. Based on this, when the medical system needs to implement new functions, the agent meeting the demands may be automatically generated through the agent platform and the input information. Further, a programming technology threshold required for the user to develop the agent is reduced, and the convenience of agent construction is improved. Moreover, since the agent may be imported into the medical system for internal operation, it may be considered that the testing of the agent may also be completed inside the medical system. Further, it is ensured that the clinical data required for the medical system to implement new functions does not need to be sent externally, thereby improving the security and privacy of clinical data. In addition, when generating and/or editing the agent based on the input information, the agent may also be a native agent exported from the medical system through the transmission unit. Further, there is no need to develop the agent meeting the demands from scratch, and only the native agent needs to be edited based on the agent platform, so as to further improve the generation efficiency of the agent. By creating or editing the agent on the agent platform and running the agent inside the medical system, it is possible to not only meet the user's demands for generating personalized and multi-functional agents but also ensure that the operation of the agent does not cause hospital information security issues.

As an example, the user's demand is to perform batch comparative analysis on segmentation results and planning results determined by the agent with the user's purely manual sketching and manual planning results in historical medical data, and a data volume including 100,000 cases of historical patient data in the medical database. The user may input the demand through the interaction layer 111 in a form of natural language. The input information may be a text, a voice, an image, or other types of information. Taking the input information as text information as an example, the information processing layer 113 may extract the time range and data type of the data from the input information, the purpose that the user intends to achieve (e.g., comparing the segmentation results and planning results determined by the agent with the user's manual sketching and manual planning results in historical medical data), the data scope for comparison (e.g., the comparison data is historical data in the medical database), and function description information such as generating segmentation results and planning results through the agent. During the interaction with the user, the agent platform 110 may further determine information such as the storage location of the data to be extracted (e.g., stored in a core database in the hospital or a specific database to facilitate subsequent data extraction operations), an algorithm to be used for result comparison, rules to be satisfied during the comparison process, and a way of presenting the comparison results, so as to fully determine a specific operational process and the algorithms, thereby improving the function description information corresponding to the execution of the above functions. The logic processing layer 114 may determine a plurality of target components as well as the execution sequence and dependency relationships (e.g., which may be represented by a pipeline layout, a functional module block diagram, or the like) of the plurality of target components based on the above function description information. Finally, the agent assembly layer 115 may determine the target components corresponding to the functional component from the preset component library, and construct the aforementioned agent based on the component relationships between the respective target components in the functional component design. At this time, after the agent is constructed, it may be imported into the medical system 320 for operation. During the operation, the agent assembly layer 115 may complete the writing of script interface codes supported by an internal medical system a hospital based on the target components that need to obtain data from the medical system 320 in a pipeline block diagram and the interface protocol of the medical system 320. Further, the data corresponding to the above input information provided by the user is obtained based on the written interface. Finally, during the operation of the agent, corresponding reports or charts may be generated based on the obtained data and the output requirements (e.g., the way of presenting the above comparison results) to display the comparison between the segmentation results and the planning results.

Merely by way of example, when obtaining data, for example, when involving the function of database data query, it is necessary to invoke an external large model to generate SQL query codes for a table structure of the medical database and embed the SQL query codes into the script. The table structure of the medical database refers to a logical design and a metadata description of each data table in the medical database.

For example, when it is necessary to obtain data of 100,000 patients from the medical database, information (e.g., fields, association relationships, and other data of each table including basic patient information, treatment data, segmentation result data, planning result data, or the like) related to the table structure of the medical database is provided to an external large model (e.g., a professional code generation large model), so that the external large model can generate appropriate SQL query statements according to the demands. For example, SQL codes for obtaining required historical segmentation, planning result data, and corresponding data generated by the new intelligent model by associating different data tables through patient IDs. Then, the generated SQL query codes are embedded into the written script to ensure that the required data can be accurately extracted from the database for subsequent comparative analysis operations.

In some embodiments, the agent may also generate corresponding interface codes for display according to the determined way of presenting the comparison results. For example, if a report needs to be generated, codes for sorting the comparison result data in a certain format and outputting the data as a text report may be written in the script. Alternatively, when presentation in the form of charts is required, corresponding drawing libraries (e.g., Matplotlib, Seaborn, or the like) may be invoked to write codes to draw bar charts, line charts, or other charts meeting the requirements according to the comparison result data, so as to ensure that the comparison results can finally be displayed in the way expected by the user.

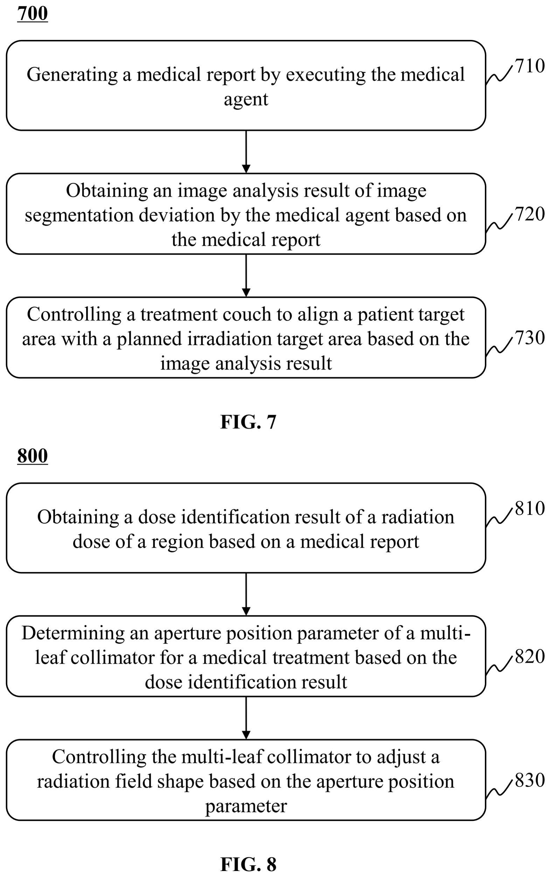

In some embodiments, the medical system 320 is further configured to generate a medical report by executing the agent. In some embodiments, the medical report includes a radiotherapy report. The medical system 320 is further configured to obtain an image analysis result of image segmentation deviation by the agent based on the medical report. The medical system 320 is further configured to control a treatment couch to align a patient target area with a planned irradiation target area based on the image analysis result. More descriptions regarding the medical system 320 generating the medical report by executing the agent may be found in the relevant parts below (e.g., FIG. 7).

In some embodiments, to control the treatment couch to align the patient target area with the planned irradiation target area based on the image analysis result, the medical system 320 is further configured to determine a three-dimensional displacement parameter and a rotation angle parameter of the treatment couch based on the image analysis result. The medical system 320 is further configured to control the treatment couch to align the patient target area with the planned irradiation target area based on the three-dimensional displacement parameter and the rotation angle parameter. More descriptions regarding the medical system 320 aligning the patient target area with the planned irradiation target area by controlling the treatment couch may be found in the relevant parts below (e.g., FIG. 7).

In some embodiments, the medical system 320 is further configured to obtain a dose identification result of a radiation dose of a region based on the medical report. The region includes a tumor target area, an organ of interest, etc. In some embodiments, the medical system 320 is further configured to determine an aperture position parameter of a multi-leaf collimator for a medical treatment based on the dose identification result. In some embodiments, the medical system 320 is further configured to control the multi-leaf collimator to adjust a radiation field shape based on the aperture position parameter. More descriptions regarding the medical system 320 controlling the multi-leaf collimator to adjust the radiation field shape through the medical report may be found in the relevant parts below (e.g., FIG. 8).

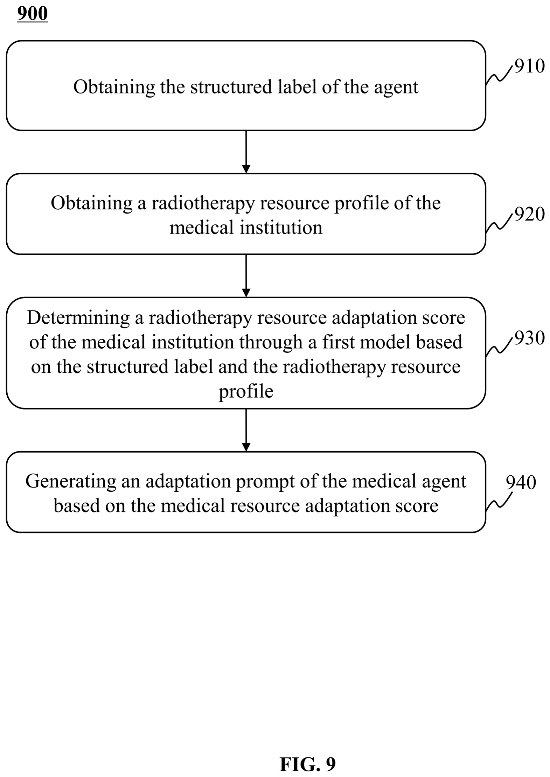

In some embodiments, the agent further includes a structured label. The structured label is configured to characterize a medical task type that the medical agent is capable of performing. In some embodiments, the medical system 320 is further configured to obtain the structured label of the agent. In some embodiments, the medical system 320 is further configured to obtain a medical resource profile of the medical institution. The medical resource profile includes a department type, an equipment configuration, and historical case data. In some embodiments, the medical system 320 is further configured to determine a medical resource adaptation score of the medical institution through a first model based on the structured label and the medical resource profile. In some embodiments, the medical system 320 is further configured to generate an adaptation prompt of the agent based on the medical resource adaptation score. More descriptions regarding the medical system 320 determining the structured label and generating the adaptation prompt of the agent may be found in the relevant parts below (e.g., FIG. 9).

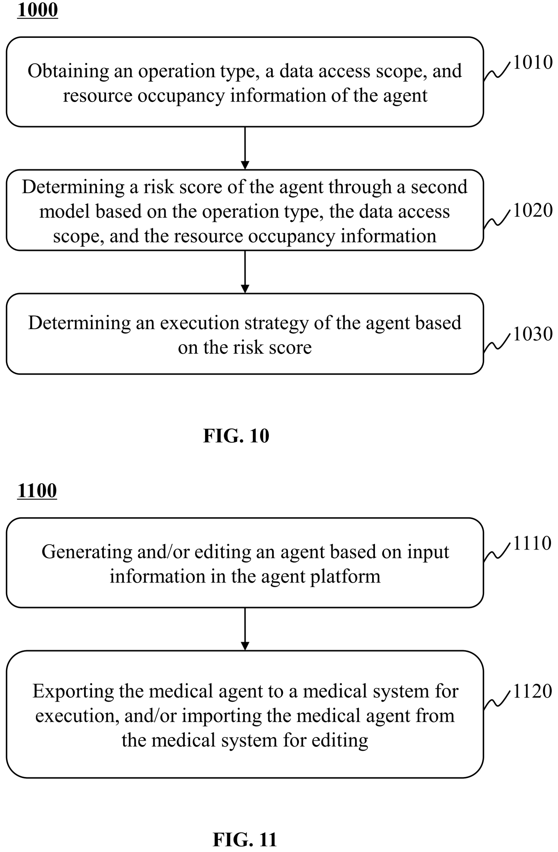

In some embodiments, the medical system 320 is further configured to obtain an operation type, a data access scope, and resource occupancy information of the agent. In some embodiments, the medical system 320 is further configured to determine a risk score of the agent through a second model based on the operation type, the data access scope, and the resource occupancy information. In some embodiments, the medical system 320 is further configured to determine an execution strategy of the agent based on the risk score. More descriptions regarding the medical system 320 determining the risk score of the agent and the execution strategy of the agent may be found in the relevant parts below (e.g., FIG. 10).

In some embodiments, the medical system 320 is further configured to determine a plurality of agents having different medical functions based on the input information of the user. In some embodiments, the medical system 320 is further configured to collaboratively execute the plurality of agents based on a preset execution sequence and a trigger condition. More descriptions regarding the medical system 320 collaboratively executing the plurality of agents may be found in the relevant parts below (e.g., FIG. 10).

In some embodiments of the present disclosure, a method for generating a medical agent (also referred to as the agent) is provided. In some embodiments, the method for generating the medical agent is executed based on the system 100 (e.g., the agent platform 110). In some embodiments, the method for generating the medical agent includes generating an agent based on input information of a user, a medical functional knowledge base, and a preset component library. More descriptions regarding generating the agent may be found in FIG. 2 and related descriptions thereof.

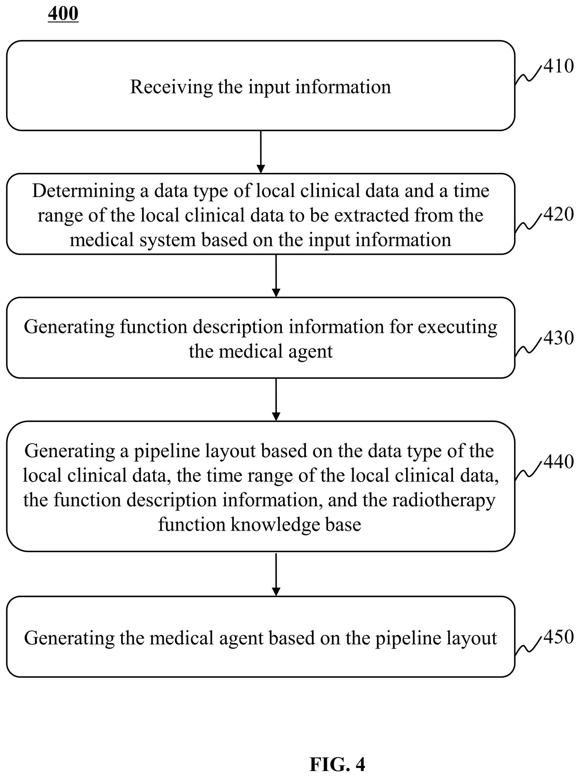

FIG. 4 is a schematic diagram illustrating an exemplary process for generating an medical agent according to some embodiments of the present disclosure. In some embodiments, the process 400 may be executed based on the system 100 (e.g., the agent platform 110). In some embodiments, the process 400 includes the following steps.

In 410, receiving input information. In some embodiments, 410 may be executed by the interaction layer 111.

In 420, determining a data type of local clinical data and a time range of the local clinical data to be extracted from the medical system based on the input information. In some embodiments, 420 may be executed by the natural language processing layer 112.

In 430, generating function description information for executing the medical agent. In some embodiments, 430 may be executed by the information processing layer 113.

In 440, generating a pipeline layout based on the data type of the local clinical data, the time range of the local clinical data, the function description information, and the medical functional knowledge base. In some embodiments, 440 may be executed by the logic processing layer 114.

In 450, generating the medical agent based on the pipeline layout and the preset component library. In some embodiments, 450 may be executed by the agent assembly layer 115.

More descriptions regarding the agent platform 110 generating the agent may be found in FIG. 2.

FIG. 5 is a schematic diagram illustrating an exemplary process for generating an medical agent according to other embodiments of the present disclosure. In some embodiments, the process 500 may be executed based on the system 100 (e.g., the agent platform 110). In some embodiments, the process 500 includes the following steps.

In 510, obtaining a plurality of target components from a preset component library.

The target component refers to a functional component selected from the preset component library. The target component is configured to complete basic tasks inside the agent. For example, the target components include data loading components, image preprocessing components, structural analysis components, indicator determination components, or the like.

In some embodiments, each of the plurality of target components is configured to execute a specific medical subtask.

The medical subtask refers to a subdivided step that can be split from an entire medical process. For example, the medical subtasks include data loading tasks, image preprocessing tasks, structural analysis tasks, indicator determination tasks, result comparison tasks, or the like. Each target component corresponds to one medical subtask.

In some embodiments, the agent assembly layer 115 in the agent platform 110 may screen components adapted to the medical subtasks from the preset component library based on nodes of the pipeline layout as matching criteria. For example, the agent assembly layer 115 may read functional identifiers corresponding to each node in the pipeline layout, and obtain a plurality of target components from the preset component library based on the functional identifiers. The functional identifier refers to a marker for identifying a function corresponding to each node in the pipeline layout.

In 520, determining a component positional relationship and an operational sequence of the plurality of target components based on the pipeline layout.

The component positional relationship refers to the logical arrangement position and association structure of each target component in the pipeline layout. The component positional relationship defines an upstream and downstream attribution between components. For example, a data loading component is located upstream of an image preprocessing component. The component positional relationship includes, but is not limited to, a parent-child relationship (e.g., a nested relationship) and a sibling relationship, and no limitation is imposed thereon. The term "upstream" refers to a component position through which data or control flow passes first in the pipeline layout. For example, if component A is located upstream of component B, it indicates that component A is executed prior to component B. An output of the component A (e.g., a processing result, intermediate data) will serve as an input of a subsequent component (e.g., the component B). The term "downstream" refers to a component position to which data or control flow arrives later in the pipeline layout. For example, if the component B is located downstream of the component A, it indicates that the component B is executed after the component A. The downstream component relies on an output of the upstream component as its own input.

In some embodiments, the agent assembly layer 115 may determine a logical position (e.g., an upstream component, a middle-stream component, a downstream component) of each target component according to an arrangement order of nodes in the pipeline layout. For example, the data loading component is located at an uppermost upstream, and the result comparison component is located at a lowest downstream, or the like.

The operational sequence refers to an execution order and a trigger condition of components determined based on connection rules of the pipeline layout. The operational sequence clarifies a data transmission path among components. For example, the operational sequence includes executing the data loading component first, then triggering the image preprocessing component, or the like.

In some embodiments, the agent assembly layer 115 may determine the operational sequence of the plurality of target components according to arrow directions in the pipeline layout. An exemplary operational sequence includes "data loading component → image preprocessing component → structural analysis component → dose determination component". In some embodiments, the agent assembly layer 115 may further identify a trigger dependency relationship between different target components. For example, structural analysis is only triggered after image preprocessing is completed.

In 530, combining the plurality of target components to generate the agent based on the component positional relationship and the operational sequence.

In some embodiments, the agent assembly layer 115 may set an execution order and a data transmission path among the plurality of target components according to the component positional relationship and the operational sequence, connecting the plurality of target components in series based on the execution order and the data transmission path, and generate the medical agent.

In some embodiments of the present disclosure, the agent platform 110 may first extract function description information for implementing demands based on the input information input by the user, and automatically generate a pipeline layout for implementing corresponding functions based on the function description information. Then, corresponding target components are determined based on the pipeline layout to automatically assemble the target components in combination with component relationships among the respective target components in the pipeline layout, thereby generating the agent. Based on this, generating the agent in the above manner does not require the user to understand programming languages or write program codes, thereby improving the generation efficiency of the agent. That is, the agent platform can, through a close cooperation and a progressive workflow of the interaction layer 111, the natural language processing layer 112, the information processing layer 113, the logic processing layer 114, and the agent assembly layer 115, finally construct a medical agent that meets the actual medical demands of the input information and has practical functions starting from the input information.

In some embodiments, the process for generating the medical agent further includes: displaying the pipeline layout in a visual interface; editing the pipeline layout based on an editing instruction of the user; performing verification on a medical function of the agent based on preset test data; and displaying a verification result in the visual interface. More descriptions regarding related content may be found in FIG. 2.

FIG. 6 is a schematic diagram illustrating an exemplary process for executing an medical agent according to some embodiments of the present disclosure. In some embodiments, the process 600 may be executed based on a medical system (e.g., the medical system 320). In some embodiments, the process 600 includes the following steps.

In 610, importing an medical agent.

In 620, executing the medical agent based on local clinical data of a medical institution.

More descriptions regarding the medical system 320 executing the medical agent may be found in FIG. 3.

FIG. 7 is a schematic diagram illustrating an exemplary process for controlling a treatment couch according to some embodiments of the present disclosure. In some embodiments, the process 700 may be executed based on a medical system (e.g., the medical system 320). In some embodiments, the process 700 includes the following operations.

In 710, generating a medical report by executing the medical agent.

In some embodiments, a medical task includes a radiotherapy task, the medical report includes a radiotherapy report. The medical report refers to a structured analysis document. For example, the medical report is a structured analysis document generated by the medical system after executing the agent. The medical report includes key information such as an image segmentation deviation, an organ dose distribution, a target area matching degree, or the like. The medical report may be presented in forms such as charts (e.g., comparison charts, dose distribution charts), texts, or the like. The image segmentation deviation refers to a spatial difference between contours of the same anatomical structure generated from an online image and a planning image. The organ dose distribution refers to a spatial distribution of radiation doses received by each point in a specific organ (or target area) in a medical plan. The target area matching degree refers to a spatial coincidence degree between an actual irradiation target area of a patient and a planned irradiation target area.

In some embodiments, the agent may generate the medical report based on local clinical data of the medical institution. For example, when input information of a user includes "generate a radiotherapy report of patient A within a time range X", the medical agent generation system 100 may generate a corresponding agent. The medical system 320 executes the agent (e.g., provides radiotherapy data of patient A within the time range X to the agent through a system access interface), and the agent generates the corresponding radiotherapy report.

In 720, obtaining an image analysis result of image segmentation deviation by the medical agent based on the medical report.

The image analysis result of image segmentation deviation refers to an analysis result obtained by the agent after comparing the online image with the planning image. The image analysis result includes physical offset data of a target area of the patient undergoing radiotherapy relative to a radiotherapy target area in an original treatment plan.

The online image refers to a real-time image of a target area and surrounding organs in the patient acquired by medical equipment (e.g., an imaging system supporting a linear accelerator) during a medical execution phase (e.g., during radiotherapy). The online image reflects a current actual body position, a target area position, and an organ morphology of the patient.

The planning image refers to a high-resolution image of the target area and surrounding organs in the patient acquired by high-precision imaging equipment (e.g., medical spiral CT) according to the medical plan during a medical planning formulation phase. The planning image is a benchmark for doctors to outline target areas, design radiation fields, and determine radiotherapy doses.

In some embodiments, the medical system 320 may extract offset data related to the image analysis result of image segmentation deviation generated by the agent from the medical report. For example, the medical system 320 may extract three-dimensional offset data (e.g., deviation data of the online image and the planning image in directions of three coordinate axes x, y, and z) between the online image and the planning image, rotation angle offset data (e.g., deviation data of a pitch angle, a roll angle, and a yaw angle between the online image and the planning image), and target area coincidence rate (e.g., a coincidence ratio between an actual irradiation target area of the patient in the online image and a planned irradiation target area in the planning image) from the medical report through methods such as semantic analysis. A coordinate system where the three coordinate axes x, y, and z are located may be preset. For example, the coordinate system where the three coordinate axes x, y, and z are located may be a DICOM image coordinate system, or the like.

In 730, controlling a treatment couch to align a patient target area with a planned irradiation target area based on the image analysis result.

The patient target area refers to an actual tumor area that receives treatment (e.g., the radiotherapy) in the patient (e.g., lung tumor tissue of a lung cancer patient).

The planned irradiation target area refers to a preset target area to be irradiated in a treatment plan (e.g., a radiotherapy plan).

In some embodiments, to control the treatment couch to align the patient target area with the planned irradiation target area based on the image analysis result, the medical system 320 may determine a three-dimensional displacement parameter and a rotation angle parameter of the treatment couch based on the image analysis result. The medical system 320 may further control the treatment couch to align the patient target area with the planned irradiation target area based on the three-dimensional displacement parameter and the rotation angle parameter.

The three-dimensional displacement parameter refers to adjustment values of the treatment couch in three linear directions of X (left-right), Y (front-back), and Z (up-down) in three-dimensional space. The three-dimensional displacement parameter is configured to correct translational offset of the patient target area (e.g., ΔX=2.3mm indicates that the treatment couch needs to be translated leftward by 2.3mm, or the like).

In some embodiments, the medical system 320 may use the three-dimensional offset data in the image analysis result as the three-dimensional displacement parameter.

The rotation angle parameter refers to rotation values of the treatment couch in three dimensions of pitch (front-back tilt), roll (left-right tilt), and yaw (horizontal rotation). The rotation angle parameter is configured to correct attitude offset (e.g., a pitch angle of 0.5° indicates that the front end of the treatment couch needs to be raised by 0.5°) of the patient target area.

In some embodiments, the medical system 320 may use the rotation angle offset data in the image analysis result as the rotation angle parameter.

In some embodiments, the medical system 320 may further perform rationality verification on the three-dimensional displacement parameter and the rotation angle parameter of the treatment couch to determine whether the three-dimensional displacement parameter and the rotation angle parameter exceed mechanical limits of the treatment couch, respectively. The mechanical limits of the treatment couch refer to maximum values of three-dimensional displacement and rotation angle of the treatment couch. If the three-dimensional displacement parameter and the rotation angle parameter of the treatment couch exceed the mechanical limits of the treatment couch, respectively, the medical system 320 may mark (e.g., re-outline the target area or adjust the treatment plan) that the offset is excessive and trigger manual adjustment by the user.

In some embodiments, the medical system 320 may further adjust the treatment couch based on the three-dimensional displacement parameter and the rotation angle parameter that have passed the rationality verification, and control the treatment couch to align the patient target area with the planned irradiation target area. Aligning the patient target area with the planned irradiation target area refers that a coincidence ratio between the patient target area in the online image and the planned irradiation target area in the planning image is greater than a preset threshold (e.g., the coincidence ratio is greater than 92%, 95%, 98%, or the like).

In some embodiments of the present disclosure, adjusting the treatment couch based on the medical report generated by the agent ensures that the adjustment precision of the treatment couch meets medical requirements, minimizes treatment errors caused by target area offset, improves treatment safety and accuracy, and makes the treatment process more intelligent and automated.

FIG. 8 is a schematic diagram illustrating an exemplary process for controlling a multi-leaf collimator to adjust a radiation field shape according to some embodiments of the present disclosure. In some embodiments, the process 800 may be executed based on a medical system (e.g., the medical system 320). In some embodiments, the process 800 includes the following steps.

In 810, obtaining a dose identification result of a radiation dose of a region based on a medical report.

The dose identification result refers to radiation dose data of irradiated regions extracted from the medical report generated by the agent. The dose identification result includes information such as actual dose values, safe tolerance thresholds, whether the dose exceeds a threshold, or the like. The dose identification result of the radiation dose of the region is configured to determine whether adjustment of the irradiation range is required.

In some embodiments, the medical system 320 may extract the dose identification result of the radiation dose of the region from the medical report through processes such as semantic analysis.

In 820, determining an aperture position parameter of a multi-leaf collimator for a medical treatment based on the dose identification result.

The aperture position parameter of the multi-leaf collimator refers to movement position coordinates of each leaf of the multi-leaf collimator. The aperture position parameter of the multi-leaf collimator is configured to determine an opening and closing degree and combined shape of the leaves of the multi-leaf collimator. The aperture position parameter of the multi-leaf collimator may determine a size and a contour of the radiation field for radiotherapy. In some embodiments, the aperture position parameter includes at least one position of at least one leaf of the multi-leaf collimator.

In some embodiments, the medical system 320 may determine regions (or patient target areas) with excessive radiation doses through the dose identification result. For example, the medical system 320 may determine regions with excessive radiation doses through the actual dose values.

In some embodiments, the medical system 320 may obtain spatial position information (e.g., extract region contour coordinates from scan data) of the regions with excessive doses. In some embodiments, the medical system 320 may determine areas to be shielded based on the spatial position information of the regions with excessive doses and radiation field shape parameters of the radiotherapy plan. For example, the radiation field shape of the radiotherapy plan includes a partial area (e.g., the edge of the radiation field covers the anterior wall of the heart) of the region with excessive dose, and the medical system 320 may determine the partial area (e.g., radiation field area coordinates corresponding to the anterior wall of the heart) of the region with excessive dose as the area to be shielded. In some embodiments, the medical system 320 may determine the opening and closing positions of the multi-leaf collimator based on the areas to be shielded, and further determine the aperture position parameter of the multi-leaf collimator. For example, for the areas to be shielded, the medical system 320 may determine the closed positions of the corresponding leaves; for the target area to be irradiated, the medical system 320 may determine that the multi-leaf collimator keeps the leaves in an open state (e.g., the leaf positions conform to the target area contour coordinates).

In 830, controlling the multi-leaf collimator to adjust a radiation field shape based on the aperture position parameter.

In some embodiments, the medical system 320 may drive the leaves of the multi-leaf collimator to move according to the aperture position parameter, control the multi-leaf collimator to adjust the radiation field shape, and form an optimized radiation field.

In some embodiments of the present disclosure, by dynamically adjusting the radiation field shape of the multi-leaf collimator, the radiation dose to the target area is ensured, while the radiation dose received by sensitive regions is reduced, radiation damage is avoided, and the safety and accuracy of a medical treatment are improved.

FIG. 9 is a schematic diagram illustrating an exemplary process for generating an adaptation prompt of an medical agent according to some embodiments of the present disclosure. In some embodiments, the process 900 may be executed based on a medical system (e.g., the medical system 320). In some embodiments, the process 900 includes the following steps.

In 910, obtaining the structured label of the agent.

In some embodiments, the agent further includes a structured label.

The structured label refers to standardized annotation information generated synchronously with the agent. The structured label is configured to characterize a medical task type applicable to the agent. For example, the structured label includes encoded information such as a disease type applicable to the agent, required equipment capabilities, a data volume requirement, and dependent components. An exemplary structured label includes "lung cancer-linear accelerator-100 cases of data-image segmentation component". It should be noted that one agent may be applicable to multiple medical tasks, so one agent may also have a plurality of structured labels.

In some embodiments, after importing the agent, the medical system 320 may automatically read the structured label in the agent data.

In 920, obtaining a medical resource profile of the medical institution.

The medical resource profile refers to a structured description of resource capabilities of the medical institution. In some embodiments, the medical resource profile of the medical institution includes a department type, an equipment configuration, historical case data, or the like.

The department type refers to a type of a medical disease department (e.g., a lung cancer, a breast cancer, a head and neck tumors) set up by the medical institution. The equipment configuration refers to a medical equipment configuration of each department. For example, the equipment configuration includes a medical equipment model (e.g., a Varian TrueBeam linear accelerator, a CBCT imaging system), an equipment quantity, an operating status, or the like. The historical case data refers to a number of historical medical cases for each disease type (e.g., 500 cases of lung cancer, 300 cases of esophageal cancer, or the like).

In some embodiments, the medical system 320 may collect and count historical medical task records in the medical institution, and periodically generate and update the medical resource profile.

In 930, determining a medical resource adaptation score of the medical institution through a first model based on the structured label and the medical resource profile.

The medical resource adaptation score refers to a quantitative indicator for measuring an executability and practicality of the medical agent in the medical institution. A higher medical resource adaptation score indicates a stronger adaptability of the agent in the medical institution.

The first model refers to a machine learning model for determining the medical resource adaptation score. For example, the first model may include a neural network model, a deep neural network model, or the like.

Inputs of the first model include the structured label and the medical resource profile, and an output includes the medical resource adaptation score.

In some embodiments, the first model may be obtained through training with first samples and first labels. In some embodiments, the medical system 320 may select historical medical records covering multiple disease types, execution effects, and resource configurations as preferred samples, use the structured labels and medical resource profiles in the preferred samples as the first samples, and use the medical resource adaptation scores scored by experts corresponding to the preferred samples as the first labels.

In 940, generating an adaptation prompt of the medical agent based on the medical resource adaptation score.

The adaptation prompt refers to natural language that provides a user with feedback on a degree of adaptation of the medical agent to the medical institution, an usage suggestion, or the medical resource adaptation score. Exemplary adaptation prompts of the agent include: "high adaptability, can be directly executed clinically", "equipment mismatch, it is recommended to try in a test environment", or the like. The adaptation prompt of the agent may be presented in the form of a pop-up window or a list note.

In some embodiments, the medical system 320 may query a preset prompt template library according to the medical resource adaptation score, generate a prompt corresponding to the medical resource adaptation score, and present the adaptation prompt in the form of a pop-up window, a list note, or the like when the user attempts to import or invoke the agent.

In some embodiments of the present disclosure, introducing the structured label and the medical resource profile for adaptability analysis implements a pre-risk assessment and adaptation recommendation mechanism during cross-institution deployment of the agent. This can effectively avoid agent execution failures and misjudgment problems caused by disease type differences, inconsistent equipment, or data missing, help users more accurately select agents matching the medical institution, and improve the stability and clinical availability of system deployment. In the embodiments of the present disclosure, the description of the agent applied to the radiotherapy process is merely an example. The agent may also be applied to other medical tasks. For example, the agent may also be applied to digital analysis of pathological sections, triage and preliminary diagnosis of clinical symptoms, surgical path planning and risk early warning, chronic disease management, or the like.

FIG. 10 is a schematic diagram illustrating an exemplary process for determining an execution strategy of an medical agent according to some embodiments of the present disclosure. In some embodiments, the process 1000 may be executed based on a medical system (e.g., the medical system 320). In some embodiments, the process 1000 includes the following steps.

In 1010, obtaining an operation type, a data access scope, and resource occupancy information of the agent.

The operation type of the agent refers to an operation category related to medical equipment control during the execution of the agent. For example, the operation type of the agent includes controlling the opening and closing of multi-leaf collimator leaves, adjusting the position and angle of a six-dimensional treatment couch, setting radiation field directions, specifying irradiation doses, controlling the on-off state of a radiation source, activating imaging acquisition equipment, or the like.

The data access scope refers to a coverage of historical data that needs to be accessed when the agent is executed. For example, the data access scope includes a count of patients, medical-involved body parts, time span (i.e., the time range of the called data), or the like. If there is a one-time call of image and planning data across multiple years and disease types of the entire hospital, the medical system 320 may identify an abnormal data access scope and prompt potential risks of data misuse, overload, or performance issues.

The resource occupancy information refers to resource requirements for executing the agent predicted based on a process structure and component characteristics of the agent. For example, the resource occupancy information includes a static estimation result such as a CPU/GPU occupancy rate, a memory usage, execution time, or the like. The resource occupancy information is configured to determine potential system load risks before executing the agent.

In some embodiments, after importing the agent, the medical system 320 may identify interfaces to be called, target data paths, and processing methods for executing the agent through statically analyzing the structured label, process script, and component configuration of the agent, thereby extracting the operation type, data access scope, and resource occupancy information corresponding to the agent.

In 1020, determining a risk score of the agent through a second model based on the operation type, the data access scope, and the resource occupancy information.

The risk score refers to a quantitative indicator for measuring potential system risks (e.g., an equipment misoperation, a data leakage, a system overload) during the execution of the agent. A higher risk score indicates a greater system risk.

The second model refers to a machine learning model for determining the risk score. For example, the second model may include a neural network model, a deep neural network model, or the like.

Inputs of the second model include the operation type, the data access scope, and the resource occupancy information of the agent, and an output includes the risk score of the agent.

In some embodiments, the second model may be obtained through training with second samples and second labels. In some embodiments, the medical system 320 may select historical agent operation records covering multiple disease types and execution effects as preferred samples. The medical system 320 may use the operation type, the data access scope, and the resource occupancy information in the preferred samples as the second samples, and use historical risk scores determined in combination with system abnormality rate, interface failure rate, or the like as the second labels. The historical risk scores may be comprehensively determined by executing the agent multiple times in a test environment according to the system abnormality rate, interface call failure rate, resource occupancy peak value, or the like during the multiple executions.

In 1030, determining an execution strategy of the agent based on the risk score.

The execution strategy of the agent refers to an operation rule of the agent formulated by the medical system according to the risk score. For example, the execution strategy of the agent includes a direct execution, an execution after approval, an execution only in a test environment, or the like.

In some embodiments, the medical system 320 may set corresponding risk level intervals according to the risk score output by the second model to determine the execution strategy of the agent. For example, when the risk score is ≥80, the execution strategy only allows operations in a test environment. As another example, when the score is between 50 and 79, the execution strategy requires approval from a doctor or an information supervisor before execution. As another example, when the score is <50, the execution strategy is direct execution.

In some embodiments of the present disclosure, by statically analyzing risk-related information of the agent, quantifying the risk score in combination with the model, and formulating differentiated execution strategies, an intelligent risk control system is constructed, which improves the controllability and clinical trustworthiness of the medical system operation.

In some embodiments, the medical system 320 may further determine a plurality of agents having different medical functions based on the input information of the user, and collaboratively execute the plurality of agents based on a preset execution sequence and a trigger condition.

The plurality of agents having different medical functions refers to a set of agents designed for different medical subtasks in the entire medical process. Each agent corresponds to one medical subtask in the entire medical process. For example, the plurality of agents having different medical functions includes a "target area image registration agent", a "dose optimization agent", a "medical report generation agent", or the like.

In some embodiments, after receiving the input information of the user, the medical system 320 may analyze a plurality of subtasks included therein (e.g., selecting patients with a certain disease type + a specific treatment method, calling an AI segmentation model to outline specific anatomical structures, extracting DVH curves and determining key parameters, or the like), and match a plurality of agents with corresponding functions from a locally deployed agent library.

The preset execution sequence refers to a temporal sequence relationship for the collaborative execution of the plurality of agents. For example, the preset execution sequence includes a linear execution (executing in sequence), a parallel execution (part of the agents executing simultaneously), a conditional branch execution (triggering corresponding agents when specific conditions are met), or the like.

The trigger condition refers to a prerequisite for starting the execution of the agent. For example, the trigger condition includes completing the execution of a previous agent, an output result meeting a preset threshold (e.g., target area coincidence rate >95%), an approval of a doctor, or the like.

In some embodiments, the medical system 320 may organize the plurality of agents into a task chain according to the set sequence and the trigger condition, automatically schedule the operation of each agent during execution, complete data transmission, state synchronization, and result integration, and realize cross-task collaborative operation.

In some embodiments of the present disclosure, by supporting automatic series connection and linked execution of a plurality of medical agents, modular splitting and process reconstruction of medical tasks are realized, an automation level and execution efficiency of the system are improved, the actual requirements of different disease types and clinical processes are flexibly adapted, and the scalability of the system is enhanced.

FIG. 11 is a schematic diagram illustrating an exemplary process for applying a medical agent according to other embodiments of the present disclosure. In some embodiments, the process 1100 may be executed based on the system 100 and/or a medical system (e.g., the medical system 320). In some embodiments, the process 1100 includes the following steps.

In 1110, generating and/or editing an agent based on input information in the agent platform.

In 1120, exporting the medical agent to a medical system for execution, and/or importing the medical agent from the medical system for editing.

The agent platform, the medical system, the manner of generating the agent, and corresponding beneficial effects may be found in the relevant parts above (e.g., FIGs. 1-10).

In some embodiments of the present disclosure, a medical system is further provided, including a computing device configured to import the agent from the agent platform and execute the agent, and/or export the agent to the agent platform for editing the agent.

The manner in which the computing device executes, imports, and/or exports the agent may all refer to the above examples, and no further explanation is provided herein.

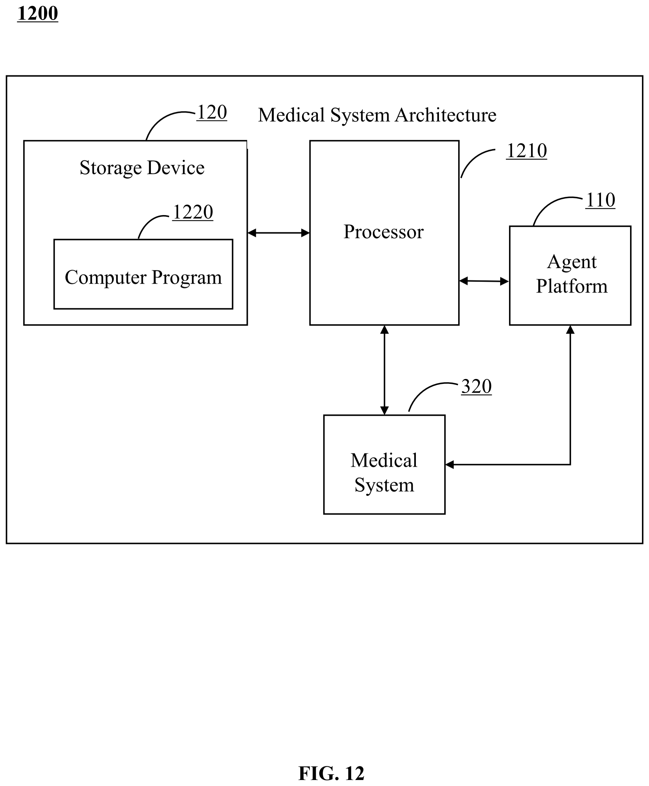

FIG. 12 is a schematic diagram of a medical system architecture according to some embodiments of the present disclosure. As shown in FIG. 12, the medical system architecture 1200 includes: the agent platform 110 and the medical system 320. The agent platform 110 is configured to generate and/or edit the agent based on input information. The medical system 320 includes a computing device. The computing device is configured to import the agent from the agent platform and execute the agent, and/or export the agent to the agent platform for editing the agent.

The medical system architecture 1200 may further include a storage device 120, a processor 1210, and a computer program 1220 (e.g., a program for a medical system upgrade method) stored in the storage device 120 and executable on the processor 1210. When the processor 1210 executes the computer program 1220, the steps in the above processes (e.g., processes 400 to 1100) are implemented to control the agent platform 110 and the medical system 320.

Merely by way of example, the computer program 1220 may be divided into one or more modules, and the one or more modules are stored in the storage device 120 and executed by the processor 1210 to implement the method for generating a medical agent, the application method, and the like provided in the embodiments of the present disclosure. The one or more modules may be a series of computer program instruction segments capable of completing specific functions, and the instruction segments are used to describe the execution process of the computer program 1220 in the medical system architecture 1200. For example, the computer program 1220 may implement the method for generating a medical agent, the application method, and the like provided in the embodiments of the present disclosure.

The medical system architecture 1200 may include, but is not limited to, the processor 1210 and the storage device 120. Those skilled in the art can understand that FIG. 12 is merely an example of the medical system architecture 1200 and does not constitute a limitation on the medical system architecture 1200. The medical system architecture 1200 may include more or fewer components than those shown in the figure, or combine certain components, or include different components (e.g., the medical system architecture may further include input/output devices, network access devices, buses, or the like).

The processor 1210 may be a central processing unit (CPU), or other general-purpose processors, digital signal processors (DSPs), application-specific integrated circuits (ASICs), off-the-shelf programmable gate arrays (FPGAs), or other programmable logic devices, discrete gate or transistor logic devices, discrete hardware components, or the like. The general-purpose processor may be a microprocessor or the processor may also be any conventional processor, or the like.

The storage device 120 may be an internal storage unit (e.g., a hard disk or a memory of the medical system architecture 1200) of the medical system architecture 1200. The storage device 120 may also be an external storage device (e.g., a removable hard disk, a smart memory card, a flash memory card, or the like configured on the medical system architecture 1200) of the medical system architecture 1200. Further, the storage device 120 may include both an internal storage unit and an external storage device of the medical system architecture 1200.

In some embodiments of the present disclosure, a computer-readable storage medium is provided, including a memory, a processor, and a computer program stored in the memory and executable on the processor. When the processor executes the computer program, the method for generating a medical agent, the application method, and the like in each of the above embodiments are implemented to control the agent platform and the medical system.

In some embodiments of the present disclosure, a computer program product is provided. When the computer program product runs on the medical system architecture, the medical system architecture is caused to execute the method for generating a medical agent, the application method, and the like in each of the above embodiments to control the agent platform and the medical system.

The basic concepts have been described above, and it is apparent to a person skilled in the art that the above detailed disclosure is intended only as an example and does not constitute a limitation of the present disclosure. While not expressly stated herein, various modifications, improvements, and amendments may be made to this disclosure by those skilled in the art. Those types of modifications, improvements, and amendments are suggested in this disclosure, so those types of modifications, improvements, and amendments remain within the spirit and scope of the exemplary embodiments of this disclosure.

## Claims

1. A medical agent generation system comprising:
an agent platform deployed on a cloud platform, wherein the agent platform is configured to generate and/or edit a medical agent based on input information of a user;
wherein the medical agent is exported to a medical system for execution, and/or the medical agent is imported from the medical system for editing.

2. The medical agent generation system of claim 1, further comprising a medical function knowledge base and a preset component library;
the medical agent is generated or edited based on the input information of the user, the medical function knowledge base, and the preset component library;
wherein the medical function knowledge base is a database that stores professional knowledge in a medical field, and the preset component library is a collection of predefined functional components.

3. The medical agent generation system of claim 1 or claim 2, wherein the agent platform generates the medical agent based on a pipeline layout, ; and the agent platform includes an interaction layer, a natural language processing layer, an information processing layer, and a logic processing layer, wherein:
the interaction layer is configured to receive the input information;
the natural language processing layer is configured to determine a data type of local clinical data and a time range of the local clinical data to be extracted from the medical system based on the input information;
the information processing layer is configured to generate function description information for executing the agent, the function description information being information capable of being understood by the medical agent; and
the logic processing layer is configured to generate the pipeline layout based on the data type of the local clinical data, the time range of the local clinical data, the function description information, and a medical function knowledge base.

4. The medical agent generation system of claim 3, wherein the agent platform further includes an agent assembly layer, and the agent assembly layer is configured to generate the medical agent based on the pipeline layout.

5. The medical agent generation system of claim 4, wherein to generate the medical agent based on the pipeline layout, the agent assembly layer is configured to:
obtain a plurality of target components from a preset component library, wherein each of the plurality of target components is configured to execute a medical subtask, the plurality of target components includes at least one of a data loading component, an image preprocessing component, a structural analysis component, or an indicator determination component, and the medical subtask is a subdivided step that is split from an entire medical process;
determine a component positional relationship and an operational sequence of the plurality of target components based on the pipeline layout, wherein the component positional relationship is a logical arrangement position and an association structure of each target component in the pipeline layout; and
combine the plurality of target components to generate the medical agent based on the component positional relationship and the operational sequence.

6. The medical agent generation system of claim 5, wherein to combine the plurality of target components to generate the medical agent based on the component positional relationship and the operational sequence, the agent assembly layer is configured to:
set an execution order and a transmission path of the local clinical data among the plurality of target components according to the component positional relationship and the operational sequence; and
generate the medical agent by connecting the plurality of target components in series based on the execution order and the transmission path.

7. The medical agent generation system of claim 6, wherein the agent assembly layer is further configured to:
determine a system access interface based on an interface protocol of the medical system, wherein the system access interface is a standardized data calling path of the medical system through which the local clinical data is retrieved from the medical system; and
extract the local clinical data based on the system access interface.

8. The medical agent generation system of any one of claims 3-7, wherein the agent platform is further configured to:
display the pipeline layout in a visual interface;
edit the pipeline layout based on an editing instruction of the user;
perform verification on the medical agent based on preset test data to obtain a verification result, wherein the preset test data is standardized case data which does not contain privacy information of a subject; and
display the verification result on the visual interface.

9. The medical agent generation system of any one of claims 3-7, wherein the medical agent is executed on a standalone device and/or in an isolated environment, and data or processes in the standalone device or the isolated environment are not subject to interference from any other device or environment.

10. A method for executing a medical agent, wherein the method is implemented by a medical system deployed at a medical institution, the method comprising:
importing a medical agent;
executing the medical agent based on local clinical data of the medical institution.

11. The method of claim 10, further comprising:
generating a medical report by executing the medical agent, wherein the medical report includes a radiotherapy report;
obtaining an image analysis result of image segmentation deviation by the medical agent based on the medical report, wherein the image segmentation deviation is a spatial difference between contours of a same anatomical structure generated from an online image and a planning image, the online image reflects a current actual body position, a radiotherapy target area position, and a radiotherapy organ morphology of a subject, the planning image is a benchmark for a user to outline a radiotherapy target area, design a radiation field, and determine a radiotherapy dose; and
controlling a treatment couch to align a patient target area with a planned irradiation target area based on the image analysis result, wherein the patient target area is an actual tumor area that receives treatment in the patient, and the planned irradiation target area is a preset target area to be irradiated in a treatment plan.

12. The method of claim 11, wherein the controlling the treatment couch to align the patient target area with the planned irradiation target area based on the image analysis result comprises:
determining a three-dimensional displacement parameter or a rotation angle parameter of the treatment couch based on the image analysis result, wherein the three-dimensional displacement parameter or the rotation angle parameter is adjustment values of the treatment couch in three-dimensional space; and
controlling the treatment couch to align the patient target area with the planned irradiation target area based on the three-dimensional displacement parameter and the rotation angle parameter.

13. The method of claim 11 or clam 12, further comprising:
obtaining a dose identification result of a radiation dose of a region based on the medical report, wherein the dose identification result is radiation dose data of the region extracted from the medical report generated by the medical agent;
determining an aperture position parameter of a multi-leaf collimator for a medical treatment based on the dose identification result, wherein the aperture position parameter includes at least one position of at least one leaf of the multi-leaf collimator; and
controlling the multi-leaf collimator to adjust a radiation field shape based on the aperture position parameter.

14. The method of any one of claims 10-13, wherein the medical agent further includes a structured label, the structured label is configured to characterize a medical task that the medical agent is capable of performing; and the method further comprising:
obtaining the structured label of the medical agent;
obtaining a medical resource profile of the medical institution, wherein the medical resource profile is a structured description of resource capabilities of the medical institution;
determining a medical resource adaptation score of the medical institution through a first model based on the structured label and the medical resource profile, wherein the medical resource adaptation score is a quantitative indicator for measuring an executability and practicality of the medical agent in the medical institution; and
generating an adaptation prompt of the medical agent based on the medical resource adaptation score, wherein the adaptation prompt is natural language that provides a user with feedback on a degree of adaptation of the medical agent to the medical institution, an usage suggestion, or the medical resource adaptation score .

15. The method of any one of claims 10-14, further comprising:
importing a plurality of medical agents having different medical functions based on input information of a user;
collaboratively executing the plurality of medical agents based on a preset execution sequence and a trigger condition.
